# EUROPEAN PATENT APPLICATION

(11) **EP 3 087 982 A1**
(43) Date of publication of application: **02.11.2016**
(21) Application number: 16167224.1
(22) Date of filing: 27.04.2016
(51) Int. Cl.: A61K 31/167, A61K 31/4174, A61K 36/534

(54) **FLAVORED ANALGESIC AND DECONGESTANT SPRAY**

(30) Priority: 01.05.2015 US 201562156081 P
(71) Applicant: Trinity ENT and Facial Aesthetics, Gilbert, AZ 85297 (US)
(72) Inventor: Vats, Veena, Gilbert, AZ Arizona 85297 (US)
(74) Representative: Hoffmann Eitle

(57) **Abstract**

The invention is a class of flavored analgesic and decongestant topical spray. The inventive liquid pharmaceutical composition sprays comprise three primary components: (1) a decongestant, (2) an analgesic, and (3) a flavorant. Liquid pharmaceutical composition sprays of the invention alleviate discomfort caused by unpleasant and unpalatable orally and nasally administered analgesic and decongestant sprays.

## Description

### BACKGROUND OF THE INVENTION

Otolaryngologists and other physicians routinely inspect patients' throats, nasal passages, and sinus cavities. Thorough examination of the surfaces of soft tissues in the nasal and throat passages requires specialized instrumentation such as an endoscope or laryngoscope. An endoscope facilitates examination of nasal cavities due to its thin and flexible nature. Optical fibers in the endoscope permit passage of light and illuminate internal surfaces for visual examination. Adequate decongestion of nasal passages is needed for thorough examination of tissue surfaces, and a topical nasal decongestant is routinely used prior to endoscopy.

Nasal endoscopy is generally considered to be a minimally invasive procedure. However, patients may be sensitive to instrumentation due to the sensitivity of mucosal membranes in the nasal and throat passages, and may be irritated by examination for a variety of other reasons. Accordingly, a topical analgesic spray applied to the nasal passage or throat - via the nostril or mouth - is utilized to desensitize tissues.

A combination decongestant and analgesic spray may be used to accomplish the dual goals of clearing the tissue surfaces of surface obstructions and desensitizing tissues. Precise amounts of decongestant and analgesic must be identified and utilized - insufficient amounts of either component leads to a more difficult exam as the membranes are not sufficiently decongested, not sufficiently anesthetized, or both. Analgesic/decongestant combination sprays are required for nearly every patient during a visit to an otolaryngologist for proper examination of the nose and mouth. Such sprays are also helpful prior to more invasive procedures such as incisions or biopsies.

However, such analgesic/decongestant sprays have an unpleasant and bitter taste and may be uncomfortable for many patients about to undergo endoscopy. It would be advantageous to develop a combination spray that accomplishes decongestion and anesthesia while remaining palatable or flavorful for the subject.

### SUMMARY OF THE INVENTION

The invention is a class of liquid pharmaceutical composition sprays designed to alleviate discomfort caused by unpleasant and unpalatable orally administered (transoral) and nasally administered (transnasal) analgesics and decongestants. The inventive sprays include a decongestant, an analgesic, and a flavorant. A spray incorporating a flavorant to mask unpleasant tastes associated with analgesic and/or decongestants would improve patient tolerance of nasal endoscopy and other procedures. Such inventive sprays may also reduce the time needed between application of the analgesic/decongestant and procedure by increasing patient comfort.

The inventive sprays may be applied via traditional nasal and throat spray application techniques (transorally and transnasally). The inventive sprays may be applied with a positive displacement pump or other atomizing spray pump that may be commonly found on otolaryngology exam carts.

It is a goal of the invention to mask unpleasant tastes associated with decongestants and analgesics. It is a goal of the invention to provide a combination decongestant and analgesic spray having palatable or flavorful characteristics.

It is a goal of the invention to achieve adequate decongestion and analgesia of a subject to permit examination of a subject's nasal or throat passages within about 120 seconds after application of the inventive sprays. It is a goal of the invention to achieve adequate decongestion and analgesia of a subject to permit examination of a subject's nasal or throat passages within about 60 seconds after application of the inventive sprays. It is a goal of the invention to achieve adequate decongestion and analgesia of a subject to permit examination of a subject's nasal or throat passages within less than about 60 seconds after application of the inventive sprays.

These and other features, aspects and advantages of the present invention will become better understood with reference to the following description and claims.

### DETAILED DESCRIPTION OF THE INVENTION

The liquid pharmaceutical composition sprays of the present invention are designed to alleviate discomfort caused by unpleasant and unpalatable orally and nasally administered analgesic and decongestant sprays. The inventive sprays comprise three primary components: (1) a decongestant, (2) an analgesic, and (3) a flavorant.

Decongestants useful in the invention include oxymetazoline, phenylephrine, ephedrine, levomethamphetamine, naphazoline, phenylephrine, phenylpropanolamine, propylhexedrine, synephrine, tetrahydrozoline, xylometazoline, pseudoephedrine, and tramazoline. Other possible decongestants useful in the invention include cafaminol, cyclopentamine, epinephrine, fenoxazoline, levonordefrin, mephentermine, metizoline, norepinephrine, tuaminoheptane, and tymazoline. One or more than one decongestant may be used in the invention. A decongestant may be selected based on its individual properties and/or effects on patients including, but not limited to, quick onset of action, short duration of effect, and ease of use.

Preferred decongestants include oxymetazoline and phenylephrine. In one embodiment the preferred decongestant is oxymetazoline; in another embodiment the preferred decongestant is phenylephrine. In one embodiment both oxymetazoline and phenylephrine are used as decongestants.

In clinical practice oxymetazoline is commonly used in 0.50 mg/mL, 0.25 mg/mL and 0.10 mg/mL concentrations. Oxymetazoline may be obtained from commercial sources as oxymetazoline hydrochloride solution 0.05% w/v and 0.025% w/v (weight per volume). Phenylephrine may be obtained from commercial sources as phenylephrine hydrochloride 1.0% w/v.

The decongestant is generally present in the liquid pharmaceutical composition at a concentration of between about 0.005% to about 1.5% w/v. The decongestant may be present in the liquid pharmaceutical composition at a concentration of between about 0.05 mg/mL to about 15.0 mg/mL. Preferably the decongestant is present in the liquid pharmaceutical composition at a concentration of between about 0.10 mg/mL and about 1.00 mg/mL, preferably between about 0.10 mg/mL and about 0.50 mg/mL, preferably between about 0.25 mg/mL and about 0.50 mg/mL. Preferably the decongestant is present in the liquid pharmaceutical composition at a concentration of between about 1.0 mg/mL and about 15.0 mg/mL, preferably between about 5.0 mg/mL and about 10.0 mg/mL, preferably between about 8.0 mg/mL and about 12.0 mg/mL.

The decongestant may be present in the inventive solution at a concentration of about 0.05 mg/mL, 0.10 mg/mL, 0.15 mg/mL, 0.20 mg/mL, 0.25 mg/mL, 0.30 mg/mL, 0.35 mg/mL, 0.40 mg/mL, 0.45 mg/mL, 0.50 mg/mL, 0.55 mg/mL, 0.60 mg/mL, 0.65 mg/mL, 0.70 mg/mL, 0.75 mg/mL, 0.80 mg/mL, 0.85 mg/mL, 0.90 mg/mL, 0.95 mg/mL, or 1.00 mg/mL. The decongestant may be present in the inventive solution at a concentration of about 0.5 mg/mL, 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.00 mg/mL, 8.5 mg/mL, 9.0 mg/mL, 9.5 mg/mL, 10.0 mg/mL, 10.5 mg/mL, 11.0 mg/mL, 11.5 mg/mL, 12.0 mg/mL, 12.5 mg/mL, 13.0 mg/mL, 13.5 mg/mL, 14.0 mg/mL, 14.5 mg/mL, or 15.0 mg/mL.

In one preferred embodiment the oxymetazoline is present in the liquid pharmaceutical composition at a concentration of about 0.50 mg/mL. In one preferred embodiment the oxymetazoline is present in the liquid pharmaceutical composition at a concentration of about 0.25 mg/mL. In one preferred embodiment the phenylephrine is present in the liquid pharmaceutical composition at a concentration of about 10.0 mg/mL. In one preferred embodiment the phenylephrine is present in the liquid pharmaceutical composition at a concentration of about 5.0 mg/mL.

Analgesics useful in the invention include local analgesics including, for example, lidocaine (xylocaine), procaine (novocaine), amethocaine, cocaine, prilocaine, bupivacaine, levobupivacaine, ropivacaine, mepivacaine, tetracaine, chloroprocaine, benzocaine, and dibucaine. Other possible analgesics include fentanyl, alfentanil, remifentanil, sufentanil, buprenorphine, butorphanol, hydromorphone, ketamine, levorphanol, meperidine, methadone, morphine, nalbuphine, oxycodone, oxymorphone, and pentazocine. One or more than one analgesic may be used in the invention. An analgesic may be selected based on its individual properties and/or effects on patients including, but not limited to, mucosal absorption or analgesic half-life.

Preferred analgesics include lidocaine, procaine, and bupivacaine. In one embodiment the preferred analgesic is lidocaine; in another embodiment the preferred analgesic is procaine; in another embodiment both lidocaine and procaine are used as analgesics.

In clinical practice lidocaine is commonly used in 20 mg/mL, 40 mg/mL and 50 mg/mL concentrations. Lidocaine may be obtained from commercial sources as lidocaine hydrochloride monohydrate solution 2% w/v, 4% w/v, and 5% w/v. In clinical practice bupivacaine is commonly used in 7.5 mg/mL and 5.0 mg/mL concentrations. In clinical practice procaine is commonly used in 10 mg/mL, 20 mg/mL, 40 mg/mL and 100 mg/mL concentrations. In clinical practice cocaine is commonly used in 40 mg/mL and 100 mg/mL concentrations.

The analgesic is generally present in the liquid pharmaceutical composition at a concentration of between about 0.1% to about 20% w/v. The analgesic may be present in the liquid pharmaceutical composition at a concentration of between about 1.0 mg/mL to about 200 mg/mL. Preferably the analgesic is present in the liquid pharmaceutical composition at a concentration of between about 10 mg/mL and about 50 mg/mL, preferably between about 20 mg/mL and about 50 mg/mL, preferably between about 20 mg/mL and about 40 mg/mL. Preferably the analgesic is present in the liquid pharmaceutical composition at a concentration of between about 10 mg/mL and about 100 mg/mL, preferably between about 20 mg/mL and about 100 mg/mL, preferably between about 40 mg/mL and about 100 mg/mL.

The analgesic may be present in the inventive solution at a concentration of about 1.0 mg/mL, 1.5 mg/mL, 2.0 mg/mL, 2.5 mg/mL, 3.0 mg/mL, 3.5 mg/mL, 4.0 mg/mL, 4.5 mg/mL, 5.0 mg/mL, 5.5 mg/mL, 6.0 mg/mL, 6.5 mg/mL, 7.0 mg/mL, 7.5 mg/mL, 8.0 mg/mL, 8.5 mg/mL, 9.0 mg/mL, 9.5 mg/mL, and 10.0 mg/mL. The analgesic may be present in the inventive solution at a concentration of about 5 mg/mL, 10 mg/mL, 15 mg/mL, 20 mg/mL, 25 mg/mL, 30 mg/mL, 35 mg/mL, 40 mg/mL, 45 mg/mL, 50 mg/mL, 55 mg/mL, 60 mg/mL, 65 mg/mL, 70 mg/mL, 75 mg/mL, 80 mg/mL, 85 mg/mL, 90 mg/mL, 95 mg/mL, 100 mg/mL, 105 mg/mL, 110 mg/mL, 115 mg/mL, 120 mg/mL, 125 mg/mL, 130 mg/mL, 135 mg/mL, 140 mg/mL, 145 mg/mL, 150 mg/mL, 155 mg/mL, 160 mg/mL, 165 mg/mL, 170 mg/mL, 175 mg/mL, 180 mg/mL, 185 mg/mL, 190 mg/mL, 195 mg/mL, and 200 mg/mL

In one preferred embodiment the liquid pharmaceutical composition contains about 10 mg/mL lidocaine; in one preferred embodiment the liquid pharmaceutical composition contains about 20 mg/mL lidocaine; in one preferred embodiment the liquid pharmaceutical composition contains about 25 mg/mL lidocaine; in one preferred embodiment the liquid pharmaceutical composition contains about 40 mg/mL lidocaine; in one preferred embodiment the liquid pharmaceutical composition contains about 50 mg/mL lidocaine. In one preferred embodiment the liquid pharmaceutical composition contains about 2.5 mg/mL bupivacaine; in one preferred embodiment the liquid pharmaceutical composition contains about 4.0 mg/mL bupivacaine; in one preferred embodiment the liquid pharmaceutical composition contains about 5.0 mg/mL bupivacaine; in one preferred embodiment the liquid pharmaceutical composition contains about 7.5 mg/mL bupivacaine. In one preferred embodiment the liquid pharmaceutical composition contains about 5.0 mg/mL procaine; in one preferred embodiment the liquid pharmaceutical composition contains about 10 mg/mL procaine; in one preferred embodiment the liquid pharmaceutical composition contains about 20 mg/mL procaine; in one preferred embodiment the liquid pharmaceutical composition contains about 40 mg/mL procaine; in one preferred embodiment the liquid pharmaceutical composition contains about 50 mg/mL procaine; in one preferred embodiment the liquid pharmaceutical composition contains about 100 mg/mL procaine. In one preferred embodiment the liquid pharmaceutical composition contains about 20 mg/mL cocaine; in one preferred embodiment the liquid pharmaceutical composition contains about 40 mg/mL cocaine; in one preferred embodiment the liquid pharmaceutical composition contains about 50 mg/mL cocaine; in one preferred embodiment the liquid pharmaceutical composition contains about 100 mg/mL cocaine.

Flavorants useful in the invention include sugar alcohols such as sorbitol, mannitol, erythritol, or xylitol, mints such as peppermint (Mentha piperita), spearmint (Mentha spicata), or menthol, sugars such as fructose, sucrose, or glucose, natural sweeteners such as honey or maple syrup, sugar substitutes and artificial sweeteners such as aspartame, stevia, sucralose, or saccharin, fruit flavorings such as cherry or watermelon, or other natural or artificial flavorants such as bubble gum, cotton candy, vanilla, chocolate, or marshmallow. One or more than one flavorant may be used in the invention. Preferred flavorants include peppermint, cherry, vanilla and sorbitol. In one embodiment the preferred flavorant is peppermint. Flavorants may have odorant properties; odorants may optionally be used in addition to flavorants.

The flavorant is generally present in a concentration of about 0.0001% to about 40% by weight of the inventive spray solution depending on the potency of flavorant and desired effect. In one embodiment the flavorant is present in a concentration from about 0.0001% to about 20%, alternatively from about from about 0.0001 % to about 10%, alternatively from about 0.0001% to about 2% and alternatively from about 0.05% to about 1.0%, all by weight of the preparation.

When an artificial sweetener is used as a flavorant, the inventive spray solution may comprise from about 0.0001% to about 5% artificial sweetener by weight. In some embodiments when an artificial sweetener is used the solution may comprise from about 0.0001% to about 3.5% artificial sweetener, from about from about 0.0001% to about 2.0% artificial sweetener, from about from about 0.0001% to about 1.0% artificial sweetener or from about 0.05% to about 1.0% artificial sweetener, all by weight of the preparation.

When a natural sweetener is used as a flavorant, the inventive spray solution may comprise from about 5% to about 40% by weight. In some embodiments when a natural sweetener is used the solution may from about 10% to about 25% natural sweetener, or from about 15% to about 20% natural sweetener by weight.

When a mint is used as a flavorant, the inventive spray solution may comprise from about 5 mg/mL to about 100 mg/mL. In one embodiment the mint flavorant is present in an amount between about 10 mg/mL and about 50 mg/mL; in one embodiment the mint flavorant is present in an amount between about 15 mg/mL and about 30 mg/mL. Peppermint may be obtained from commercial sources as peppermint oil solution 1200 mg/mL and 1000 mg/mL. Peppermint oil may comprise about 5% to about 55% methanol and from about 10% to about 50% menthone. In a preferred embodiment the peppermint is present in the inventive spray in an amount of about 20-25 mg/mL. In a preferred embodiment the peppermint is present in the inventive spray in an amount of about 23 mg/mL. Peppermint is a preferred flavorant for its pleasant taste as well as for its analgesic properties.

Spray solutions of the invention may be prepared by conventional mixing methods. In one embodiment a liquid decongestant is mixed with a liquid analgesic, to which a flavorant is then added. In a preferred embodiment a solution of about 1 mL is prepared by mixing about 0.5 mL lidocaine (4% lidocaine hydrochloride monohydrate w/v) with about 0.5 mL oxymetazoline (0.05% oxymetazoline hydrochloride solution w/v) to which about 3 drops of peppermint oil (60 mg/drop) was added. The components are mixed vigorously to ensure thorough distribution prior to administration to a subject.

The inventive sprays may be delivered by a metered pump or via a motorized atomizer. A motorized atomizer is generally comprised of a motor coupled to tubing and an atomizing nozzle. The inventive solution may be placed in a glass container with straw and cap and coupled to the atomizer. The motor generates a constant flow of air through the tubing permitting the solution to be drawn up into the straw and sprayed out of the nozzle in a fine mist.

In a manual pump embodiment, each spray may deliver a dose of between about 50 microliters to about 200 microliters. In one embodiment each spray may delivers a dose of about 100 microliters. In one embodiment each spray delivers a dose of about 125 microliters of the liquid pharmaceutical composition. In one embodiment each spray delivers a dose of about 0.025 mg oxymetazoline, about 2.0 mg lidocaine, and about 2.3 mg peppermint oil. In one embodiment each spray delivers a dose of about 0.05 mg oxymetazoline, about 4.0 mg lidocaine, and about 2.3 mg peppermint oil.

Slightly acidic or neutral solutions are preferred for the liquid pharmaceutical compositions of the invention. The pH of the inventive compositions may be between about 4.5 and 7.0. The pH of the inventive compositions may be about 4.5, about 5.0, about 5.5, about 6.0, about 6.5, or about 7.0. In one embodiment the pH of the liquid pharmaceutical composition is between about 5.5 and 6.5. In one embodiment the pH of the liquid pharmaceutical composition is about 5.5; in one embodiment the pH of the liquid pharmaceutical composition is about 6.0; in one embodiment the pH of the liquid pharmaceutical composition is about 6.5.

### FURTHER EMBODIMENTS

Liquid pharmaceutical compositions of the invention may optionally include antimicrobial components or preservatives. These may be present in the liquid pharmaceutical composition in an amount of about 0.001% to about 0.050% by weight. Examples of optional components include benzyl alcohol, phenylethyl alcohol, benzalkonium chloride, methyl parabens, ethyl parabens, propyl parabens and butyl parabens.

Additional optional components may be included such as buffers, surfactants, and emulsifiers. Optional components may include citric acid, sodium phosphate, EDTA (ethylenediaminetetraacetic acid), polyoxyethylene (20) sorbitan monooleate, polyethylene glycol, and polysorbate 80.

### EXAMPLES

### EXAMPLE 1

In this example, eight (8) exemplary sprays were prepared using varying amounts of oxymetazoline, lidocaine, and peppermint oil. In each preparation, the oxymetazoline was sourced from a 0.05% oxymetazoline hydrochloride solution; the lidocaine was sourced from a 4% lidocaine hydrochloride monohydrate solution; and the peppermint was sourced from a 60 mg/drop solution. The eight solutions are detailed in the following table:

| Preparation | Oxymetazoline (mL) | Lidocaine (mL) | Peppermint (drops) |
|---|---|---|---|
| 1 | 0.5 | 0.5 | 1 |
| 2 | 1 | 1 | 1 |
| 3 | 2 | 2 | 1 |
| 4 | 2 | 2 | 2 |
| 5 | 3 | 3 | 2 |
| 6 | 3 | 3 | 3 |
| 7 | 4 | 4 | 3 |
| 8 | 4 | 4 | 4 |
| control | 5 | 5 | 0 |

Each preparation was prepared by mixing the oxymetazoline with lidocaine and subsequently adding the peppermint. A control solution was prepared by mixing 5 mL oxymetazoline (0.05% w/v) with 5 mL lidocaine (4% w/v).

The control solution without flavorant was reported to have a bitter and unpleasant taste. Preparation 1 was reported as having a peppermint flavor that was too strong and overpowering. Preparations 2, 4, 6, and 8 were also reported as having a peppermint flavor that was too strong and overpowering though the lidocaine flavor was masked. Preparations 3 and 5 were reported as not having sufficient peppermint as the lidocaine could still be tasted. Preparation 7 was reported to have the most favorable characteristics where the peppermint masked the lidocaine and had a pleasant taste.

### ADDITIONAL EXAMPLES

1. A liquid pharmaceutical composition comprising
   (a) a decongestant,
   (b) an analgesic, and
   (c) a flavorant.
2. A liquid pharmaceutical composition of Example Composition 1 wherein said decongestant comprises oxymetazoline.
3. A liquid pharmaceutical composition of Example Composition 1 wherein said analgesic comprises lidocaine.
4. A liquid pharmaceutical composition of Example Composition 2 wherein said analgesic comprises lidocaine.
5. A liquid pharmaceutical composition of Example Composition 1 wherein said flavorant comprises peppermint.
6. A liquid pharmaceutical composition of Example Composition 2 wherein said flavorant comprises peppermint.
7. A liquid pharmaceutical composition of Example Composition 3 wherein said flavorant comprises peppermint.
8. A liquid pharmaceutical composition of Example Composition 4 wherein said flavorant comprises peppermint.
9. A liquid pharmaceutical composition of Example Composition 2 wherein said oxymetazoline is present in a concentration of between about 0.25 and about 0.50 mg/mL.
10. A liquid pharmaceutical composition of Example Composition 3 wherein said lidocaine is present in a concentration of between about 20 and 40 mg/mL.
11. A liquid pharmaceutical composition of Example Composition 5 wherein said peppermint is present in a concentration of about 20 to about 25 mg/mL.
12. A liquid pharmaceutical composition of Example Composition 2 wherein said oxymetazoline is present in a concentration of about 0.25 mg/mL.
13. A liquid pharmaceutical composition of Example Composition 3 wherein said lidocaine is present in a concentration of about 20 mg/mL.
14. A liquid pharmaceutical composition of Example Composition 5 wherein said peppermint is present in a concentration of about 23 mg/mL.
15. A liquid pharmaceutical composition of Example Composition 2 wherein said oxymetazoline is present in a concentration of about 0.50 mg/mL.
16. A liquid pharmaceutical composition of Example Composition 3 wherein said lidocaine is present in a concentration of about 40 mg/mL.
17. A liquid pharmaceutical composition of Example Composition 1 wherein said flavorant is present in an amount sufficient to mask the flavor of said analgesic.
18. A liquid pharmaceutical composition of Example Composition 1 wherein said analgesic is present in an amount sufficient to provide a subject with analgesia.
19. A liquid pharmaceutical composition of Example Composition 1 wherein said decongestant is present in an amount sufficient to effect nasal decongestion.
20. A liquid pharmaceutical composition comprising:
   between about 0.25 mg/mL and about 0.50 mg/mL oxymetazoline;
   between about 20 mg/mL and about 40 mg/mL lidocaine; and
   between about 20 mg/mL to about 25 mg/mL peppermint.
21. A liquid pharmaceutical composition of Example Composition 20 comprising
   about 0.25 mg/ml oxymetazoline;
   about 20 mg/ml lidocaine; and
   about 23 mg/ml peppermint.
22. A liquid pharmaceutical composition of Example Composition 20 comprising about 0.50 mg/ml oxymetazoline;
   about 40 mg/ml lidocaine; and
   about 23 mg/ml peppermint.
23. A liquid pharmaceutical composition comprising:
   between about 0.05 mg/mL to about 15.0 mg/mL decongestant;
   between about 1.0 mg/mL to about 200 mg/mL analgesic; and
   a flavorant.
24. A liquid pharmaceutical composition of Example Composition 23 comprising:
   between about 0.25 mg/mL and about 0.50 mg/mL oxymetazoline; and
   between about 20 mg/mL and about 40 mg/mL lidocaine.
25. A liquid pharmaceutical composition of Example Composition 23 wherein said flavorant is peppermint.
26. A liquid pharmaceutical composition of Example Composition 24 comprising about 23 mg/mL peppermint.
27. A method of improving the tolerability of an analgesic in a subject following nasal administration of a liquid pharmaceutical composition of an analgesic and decongestant, which method comprises adding a flavorant to said composition prior to said administration.
28. A method of Example Method 27 where said flavorant is present in an amount between about 1 mg/mL and about 40 mg/mL.
29. A method of administering an analgesic to a subject requiring an analgesic, said method comprising nasally administering to said subject a therapeutically effective amount of the composition of Example Composition 1.
30. A method of administering an analgesic to a subject requiring an analgesic, said method comprising nasally administering to said subject a therapeutically effective amount of the composition of Example Composition 20.
31. A method of administering an analgesic to a subject requiring an analgesic, said method comprising nasally administering to said subject a therapeutically effective amount of the composition of example composition 23.
32. A method of administering an analgesic to a subject requiring an analgesic, said method comprising orally administering to said subject a therapeutically effective amount of the composition of Example Composition 1.
33. A method of administering an analgesic to a subject requiring an analgesic, said method comprising orally administering to said subject a therapeutically effective amount of the composition of Example Composition 20.
34. A method of administering an analgesic to a subject requiring an analgesic, said method comprising orally administering to said subject a therapeutically effective amount of the composition of Example Composition 23.
35. A liquid pharmaceutical composition of Example Composition 1 wherein said composition is in the form of a nasal spray.
36. A liquid pharmaceutical composition of Example Composition 1 having a pH of between about 4.5 to about 7.0.
37. A liquid pharmaceutical composition of Example Composition 1 wherein said flavorant comprises cherry.
38. A liquid pharmaceutical composition of Example Composition 23 wherein said flavorant comprises cherry.

In view of the above, it will be seen that the several advantages of the disclosure are achieved and other advantageous results attained. As various changes could be made in the above devices and methods without departing from the scope of the disclosure, it is intended that all matter contained in the above description and shown in the accompanying drawings shall be interpreted as illustrative and not in a limiting sense.

While various embodiments have been described above, it should be understood that such disclosures have been presented by way of example only, and are not limiting. Thus, the breadth and scope of the subject methods, devices, and systems should not be limited by any of the above-described exemplary embodiments, but should be defined only in accordance with the following claims and their equivalents.

Having now fully described the subject methods, devices, and systems, it will be understood by those of ordinary skill in the art that the same can be performed within a wide and equivalent range of conditions, formulations and other parameters without affecting their scope or any embodiment thereof. All cited patents, patent applications and publications are fully incorporated by reference in their entirety.

## Claims

1. A liquid pharmaceutical composition comprising
(a) a decongestant,
(b) an analgesic, and
(c) a flavorant.

2. A liquid pharmaceutical composition according to claim 1 wherein said decongestant comprises oxymetazoline.

3. A liquid pharmaceutical composition according to claim 1 or 2 wherein said analgesic comprises lidocaine.

4. A liquid pharmaceutical composition according to any of claims 1, 2, or 3 wherein said flavorant comprises peppermint.

5. A liquid pharmaceutical composition of claim 2 wherein said oxymetazoline is present in a concentration of between about 0.25 and about 0.50 mg/mL.

6. A liquid pharmaceutical composition of claim 3 wherein said lidocaine is present in a concentration of between about 20 and 40 mg/mL.

7. A liquid pharmaceutical composition of claim 1 wherein said flavorant is present in an amount sufficient to mask the flavor of said analgesic.

8. A liquid pharmaceutical composition of claim 1 wherein said analgesic is present in an amount sufficient to provide a subject with analgesia.

9. A liquid pharmaceutical composition of claim 1 wherein said decongestant is present in an amount sufficient to effect nasal decongestion.

10. A liquid pharmaceutical composition comprising:
between about 0.25 mg/mL and about 0.50 mg/mL oxymetazoline;
between about 20 mg/mL and about 40 mg/mL lidocaine; and
between about 20 mg/mL to about 25 mg/mL peppermint.

11. A liquid pharmaceutical composition of claim 10 comprising
about 0.25 mg/ml oxymetazoline;
about 20 mg/ml lidocaine; and
about 23 mg/ml peppermint.

12. A liquid pharmaceutical composition of claim 10 comprising
about 0.50 mg/ml oxymetazoline;
about 40 mg/ml lidocaine; and
about 23 mg/ml peppermint.

13. A liquid pharmaceutical composition comprising:
between about 0.05 mg/mL to about 15.0 mg/mL decongestant;
between about 1.0 mg/mL to about 200 mg/mL analgesic; and
a flavorant.

14. A liquid pharmaceutical composition of claim 13 comprising:
between about 0.25 mg/mL and about 0.50 mg/mL oxymetazoline; and
between about 20 mg/mL and about 40 mg/mL lidocaine.

15. A liquid pharmaceutical composition of any of claims 13 and 14 wherein said flavorant is peppermint.
